# FASCICULE DE BREVET EUROPEEN

(11) **EP 0 965 315 B1**
(45) Date de publication et mention de la délivrance du brevet: **29.12.2004**
(21) Numéro de dépôt: 99401422.3
(22) Date de dépôt: 11.06.1999
(51) Int. Cl.: A61F 9/013, A61F 2/14

(54) **Chambre artificielle pour prélèvement d'un greffon de cornée**
Künstliche Kammer zur Entnahme eines Kornea-Transplantats
Artificial chamber for the excision of a cornea graft

(30) Priorité: 19.06.1998 FR 9807775
(43) Date de publication de la demande: 22.12.1999
(73) Titulaire: Moria SA, 92160 Antony (FR)
(72) Inventeur: Duprat, Alain, 31120 Roquettes (FR)
(74) Mandataire: Robert, Jean-Pierre

(56) Documents cités:
- FR-A- 2 712 184
- US-A- 4 738 401
- US-A- 4 865 033
- US-A- 4 982 969
- DATABASE WPI Section PQ, Week 9422 Derwent Publications Ltd., London, GB; Class P32, AN 94-181063 XP002095602 & SU 1 801 422 A (VOLG MED INST), 15 mars 1993 (1993-03-15)

## Description

La présente invention concerne une chambre artificielle destinée à recevoir un disque cornéoscléral prélevé sur un donneur en vue d'un prélèvement par sa face épithéliale pour former un greffon.

Au cours de ces dernières années des progrès ont été réalisés dans la mise au point des liquides de conservation des cornées prélevées destinées à être utilisées pour la greffe. Actuellement, un disque comportant la cornée entourée d'un anneau scléral d'un à deux millimètres est mis dans un liquide de conservation et peut être utilisé quelques jours plus tard.

La découpe du greffon est réalisée au moyen d'un montage, dit de chambre artificielle, qui bride la cornée en la pinçant au niveau de l'anneau scléral et qui présente des moyens de support d'un outil de découpe appelé kératome. Ce kératome peut être de deux types, trépan ou rabot, selon que l'opérateur souhaite réaliser une découpe perforante (c'est-à-dire sur toute l'épaisseur de la cornée) ou au contraire une découpe lamellaire sur une épaisseur déterminée de la cornée. La chambre artificielle permet de rigidifier la cornée par pressurisation au moyen d'un sérum physiologique ou d'air, ceci permettant une découpe par la face antérieure (épithéliale) dans des conditions très voisines de celles existantes lors d'un prélèvement de la cornée malade à remplacer.

Plusieurs structures de chambres artificielles ont déjà été proposées. On connaît notamment du brevet français n° 2 712 184 une chambre artificielle comportant un pied, un support de cornée porté par le pied, ayant un axe vertical et possédant une tête présentant une face supérieure de support sur laquelle débouche au moins un orifice d'alimentation, et une bague de bridage mobile en translation par rapport au support de cornée suivant l'axe de celui-ci entre une position de bridage dans laquelle sa face supérieure pince le disque cornéoscléral contre la bague de bridage et une position de libération dans laquelle sa face supérieure est écartée de la bague de bridage.

Un but de l'invention est le proposer un système de chambre artificielle ergonomique permettant une manipulation, un démontage et une stérilisation aisée, tout en étant de fabrication simple et peu coûteuse.

Selon l'invention, on propose une chambre artificielle pour un disque cornéoscléral en vue d'un prélèvement de greffon, comportant un pied, un support de cornée porté par le pied, ayant un axe vertical et possédant une tête présentant une face supérieure de support sur laquelle débouche au moins un orifice d'alimentation, et une bague de bridage du disque cornéoscléral sur le support de cornée, dans laquelle la bague de bridage est portée par le pied, characterisé en ce que la bague de bridage est fixe tandis que le support de cornée est monté sur le pied pour coulisser suivant l'axe vertical du support de cornée et que des moyens d'actionnement sont prévus pour faire coulisser le support de cornée.

Dans un premier mode de réalisation les moyens d'actionnement du support de cornée sont constitués par un système vis-écrou, le support de cornée possédant une partie filetée formant vis qui est arrêtée en rotation par rapport au pied et qui coopère avec un écrou monté sur le pied en rotation libre sans translation selon l'axe du support de cornée.

Dans un second mode de réalisation, plus perfectionné, les moyens d'actionnement du support de cornée sont constitués par un vérin double effet. Le chirurgien ou l'un des ses assistants peut ainsi piloter de façon commode et précise le déplacement du support de cornée. Le vérin peut en particulier être piloté au moyen d'un organe de pilotage adéquat tel qu'une pédale laissant au chirurgien les mains libres.

Selon une caractéristique avantageuse de l'invention, la bague de bridage est montée de façon amovible sur le pied.

Avantageusement alors, la fixation amovible de la bague de bridage sur le pied est réalisée au moyen d'un système à baïonnette.

Avantageusement encore, lorsque l'organe d'actionnement du support de cornée est réalisé sous la forme d'un système vis-écrou, la bague de bridage est reliée au pied par l'intermédiaire d'une partie de liaison qui entoure l'écrou d'actionnement du support de cornée et qui est ajourée pour permettre un actionnement manuel de cet écrou.

Selon une autre caractéristique avantageuse de l'invention, la bague de bridage est portée par le bord supérieur d'une couronne qui coiffe la tête du support de cornée.

La couronne présente un filetage extérieur sur lequel est engagé un écrou formant une butée réglable en hauteur pour un accessoire de kératotomie venant coiffer la couronne de bridage.

On pourra alors avantageusement prévoir que la couronne présente un filetage extérieur sur lequel est engagé un écrou formant une butée réglable en hauteur pour un accessoire de kératotomie venant coiffer la couronne de bridage.

Avantageusement alors l'accessoire de kératotomie est équipé d'un organe de son verrouillage sur la couronne de bridage.

D'autres caractéristiques et avantages de l'invention apparaîtrons à la lecture de la description qui suit de modes de réalisation particuliers donnés à titre d'exemples non limitatifs.

Il sera fait référence aux dessins en annexe, parmi lesquels
- la figure 1 est une vue axiale d'une chambre artificielle selon premier mode de réalisation conforme à l'invention ;
- figure 2 une vue analogue à la figure 1 illustrant un second mode de réalisation conforme à l'invention.

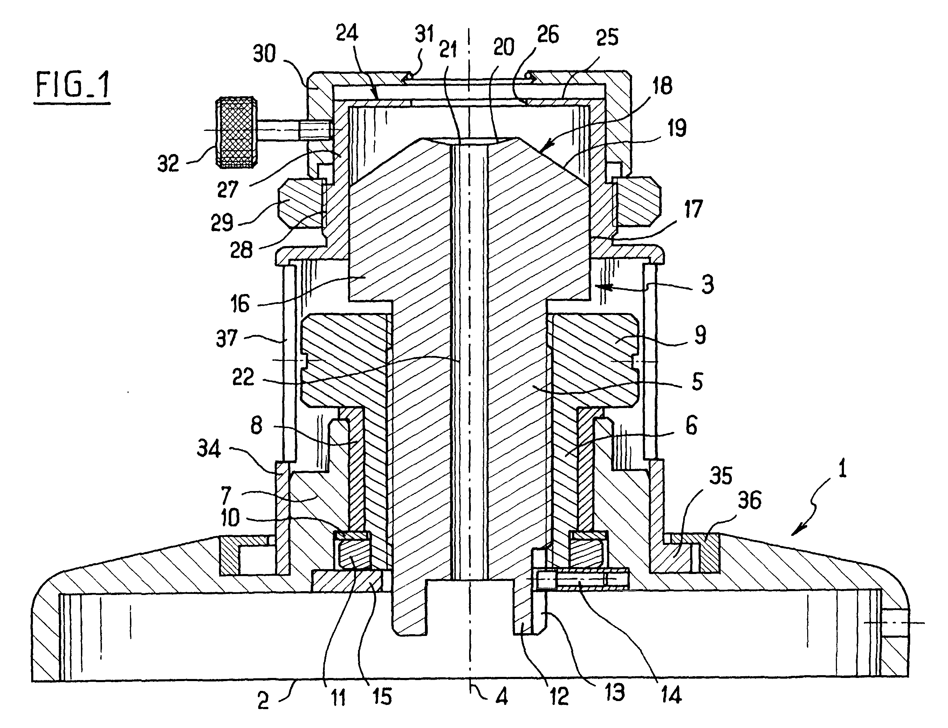

En référence à la figure 1, une chambre artificielle conforme à l'invention comporte un pied 1 présentant une face inférieure 2 plane, sensiblement horizontale.

Un support de cornée 3 de forme cylindrique étagée d'axe vertical 4 est monté sur le pied 1 pour coulisser suivant son axe vertical 4. Le montage coulissant du support de cornée 3 sur le pied 1 et sa commande sont réalisés par l'intermédiaire d'un système vis-écrou. Le support de cornée 3 possède ainsi une partie médiane filetée 5 dont le filetage extérieur coopère avec un écrou 6 monté sur le pied 1 en rotation libre sans translation selon l'axe 4.

Plus précisément, le support 1 présente un palier 7 d'axe 4 qui est équipé d'un coussinet 8 dans lequel est reçu en rotation libre l'écrou 6. L'immobilisation axiale de l'écrou est assurée par des butées associées à l'écrou de part et d'autre du coussinet 8. En l'espèce, l'écrou 6 possède à sa partie supérieure une partie annulaire élargie 9 qui permet un entraînement en rotation manuelle de l'écrou 6 et qui vient par ailleurs en butée contre l'extrémité supérieure du coussinet 8. A son extrémité inférieure, l'écrou 6 est équipé d'une rondelle de butée 10 verrouillée contre un épaulement de l'écrou 6 par un écrou d'arrêt 11.

Pour empêcher l'entraînement en rotation du support de cornée 3 par l'écrou 6, le support de cornée 3 est indexé au pied 1. A cet effet, le support de cornée 3 est pourvu à son extrémité inférieure 12 d'une rainure 13 parallèle à l'axe 4, dans laquelle est engagé un pion d'indexation 14 qui est logé dans un insert 15 en forme de rondelle rapporté sur le pied 1 à l'extrémité inférieure du palier 7.

A sa partie supérieure, le support de cornée 3 possède une tête 16 sensiblement plus large que sa partie filetée 5. Cette tête 16 présente une face latérale cylindrique 17 et une face supérieure de support 18. La face supérieure 18 se décompose en deux parties : une partie périphérique tronconique 19 et une partie centrale concave 20 centrée sur l'axe 4 et sur laquelle débouche un orifice d'alimentation 21 formé par l'extrémité supérieure d'un canal d'alimentation 22 traversant de part en part le support de cornée 3 suivant l'axe 4.

Un chapeau de bridage 24 fixe et porté par le pied 1 est disposé à l'aplomb de la face supérieure 18 du support de cornée 3, de façon à réaliser le bridage de la périphérie du disque cornéoscléral lorsque celui-ci est placé sur la face supérieure 18 du support de cornée 3 et que le support de cornée 3 est relevé en position de bridage.

Plus précisément, ce chapeau de bridage 24 possède une bague de bridage 25 en forme de disque évidé en son centre et présentant un bord intérieur 26 de forme circulaire centré sur l'axe 4. Le diamètre du bord 26 est légèrement supérieur à celui de la partie central concave 20 de la face supérieure 18 du support de cornée 3 de sorte qu'en position de bridage le bord de intérieur 26 de la bague de bridage 25 vient "mordre" la périphérie du disque cornéoscléral autour de la partie centrale concave de la face de support 18.

Le chapeau de bridage 24 possède en outre, en dessous de la bague de bridage 25, une partie de centrage 27 en forme de couronne cylindrique dont la face intérieure épouse la face latérale cylindrique 17 de la tête 16 du support de cornée 3. Cette couronne 27 coiffe la tête 16 du support de cornée 3 pour un meilleur centrage de la bague de bridage 25 par rapport à l'axe 4 du support de cornée 3.

En outre, la couronne 27 présente un filetage extérieur 28 sur lequel est engagé un écrou 29 formant une butée réglable en hauteur pour un accessoire de kératotomie 30 en forme de chapeau venant coiffer le chapeau de bridage 24. Cet accessoire de kératotomie 30 est ici un support de guidage de kératome à rainures en queue d'aronde 31. Mais il sera également possible de disposer sur le chapeau de bridage 24, avant l'opération de kératotomie en elle-même, un accessoire similaire à coupelle transparente permettant de mesurer le diamètre du greffon à découper.

Dans l'exemple illustré, l'accessoire 30 est de plus équipé d'une vis transversale de verrouillage 32 qui peut être serrée contre la face latérale extérieure de la couronne 27 pour assurer l'immobilisation de l'accessoire de kératotomie 30.

Le bord inférieur de la couronne 27 est reliée au pied 1 par l'intermédiaire d'une partie de liaison 34 qui entoure l'écrou d'actionnement 6 et le pallier 7 et qui est pourvue à son extrémité inférieure d'une collerette 35 coopérant avec une collerette conjuguée 36 associée au pied 1 pour former un système à baïonnette de fixation amovible du chapeau 24 sur le pied 1.

Cette partie de liaison 34 est ajourée et présente des ouvertures de passage 37 permettant un accès direct à la partie annulaire d'actionnement 9 de l'écrou 6 pour une manipulation aisée de ce dernier.

L'utilisation de la chambre artificielle qui vient d'être décrite se déroule de la manière suivante. Le chapeau de bridage 24 étant dégagé du support de cornée 3 pour permettre un accès direct à la face supérieure 18 de celui-ci, un disque cornéoscléral est disposé au centre de la face 18, en recouvrement de la partie centrale concave 20 et en débordement partiel sur la partie périphérique tronconique 19. Le chapeau de bridage 24 est remis en place en venant partiellement coiffer la tête 16 du support de cornée 3. La collerette 35 est engagée en correspondance dans la collerette 36 et le chapeau de bridage 24 est entraîné en rotation d'une portion de tour (par exemple un quart de tour) pour un verrouillage rapide et sûr, sur le pied 1.

Le support de cornée 3 étant initialement en position basse de libération, c'est-à-dire dans une position dans laquelle sa face supérieure 18 est écartée de la bague de bridage 25, l'écrou 6 est actionné en rotation par l'intermédiaire de sa partie annulaire de manipulation 9 de façon à faire translater le support de cornée 3 vers le haut jusqu'à ce qu'il parvienne en position de bridage de sa face supérieure 18 contre le bord 26 de la bague de bridage 25. La périphérie du disque cornéoscléral est alors pincée entre le bord 26 et la partie tronconique 19 de la face supérieure 18 du support de cornée 3. L'effort de pincement peut être réglé en serrant plus ou moins fortement l'écrou 9 en fin de course.

Le volume étanche qui est alors formé entre le disque cornéoscléral et la partie central concave 20 de la face supérieure 18 du support de cornée constitue la chambre artificielle proprement dite. Le canal d'alimentation 22 étant raccordé à un dispositif d'alimentation pneumatique ou hydraulique, cette chambre artificielle est mise sous pression pour simuler la pression interne de l'oeil et faire prendre au disque cornéoscléral sa forme convexe naturelle. Le disque cornéoscléral ainsi mis en forme par pressurisation fait saillie au-dessus de la bague de bridage 25 et est ainsi parfaitement positionné pour sa kératotomie.

Le positionnement relatif du kératome (dont seul le support de guidage 30 est représenté à la figure 1) peut être ajusté en modifiant la position de l'écrou de butée 29. Ce réglage en hauteur conditionne le degré d'aplanissement du disque cornéoscléral lors de la kératotomie et donc le diamètre du greffon prélevé. La vis de verrouillage 32 permet d'immobiliser l'accessoire 30 après ajustement de sa position.

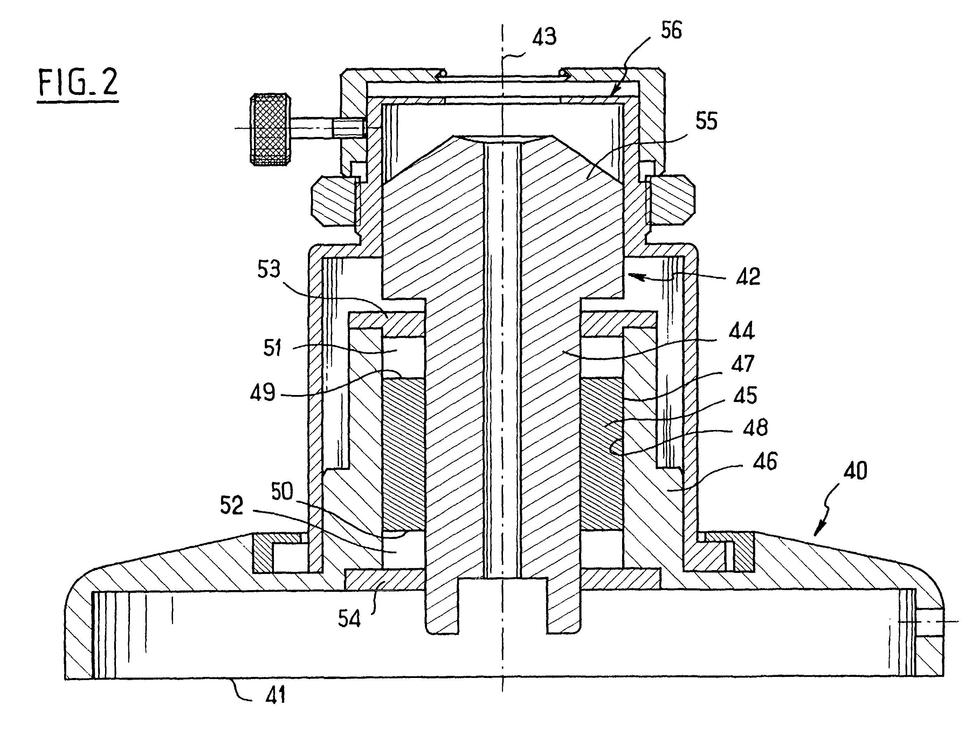

A la figure 2, on a représenté un second mode de réalisation d'une chambre artificielle conforme à l'invention.

Cette chambre comporte comme précédemment un pied 40 présentant une face inférieure 41 plane, sensiblement horizontale.

Un support de cornée 42 de forme cylindrique étagé d'axe vertical 43 est monté sur le pied 40 pour coulisser suivant son axe vertical 43. A la différence du mode de réalisation précédemment décrit, le montage coulissant du support de cornée 42 sur le pied 1 et sa commande sont réalisés au moyen d'un système de vérin double effet, pneumatique ou hydraulique. Le support de cornée 42 possède ainsi une partie inférieure 44 qui constitue la tige d'un vérin dont le corps est formé par un fourreau cylindrique 46 d'axe 43 ménagé sur le pied 40. Plus précisément, la tige 44 est pourvu d'un épaulement 45 qui présente une face latérale 47 montée coulissante dans le fourreau 46 dont la face intérieure cylindrique 48 est sensiblement de même diamètre. Eventuellement, l'épaulement 45 peut être pourvu d'un joint pour renforcer son étanchéité avec le fourreau 46. Deux flasques 53,54 rapportés sur les extrémités supérieure et inférieure du fourreau 46 assurent une fermeture étanche du fourreau 46 avec les parties supérieure et inférieure de la tige 44, de part et d'autre de l'épaulement 45. L'épaulement 45 présente deux faces radiales actives supérieure et inférieure 49, 50 qui délimitent avec le fourreau 46, les flasques 53, 54 et la tige 44 des chambres supérieure et inférieure 51, 52 qui sont alimentées en gaz ou en liquide par un circuit d'alimentation et de pilotage de type classique (non représenté aux figures).

Ainsi, le relèvement du support de cornée 42 vers sa position de bridage est obtenu en mettant sous pression la chambre inférieure 52. Inversement, l'abaissement du support de cornée 42 vers la position de libération est obtenu en mettant sous pression la chambre supérieure 51. Le pilotage de ce système de vérin peut être réalisé au moyen d'un organe de pilotage à pédale qui présente l'avantage de laisser à l'opérateur les mains libres. En outre, l'utilisation d'un système à vérin offre l'avantage de permettre un réglage commode et précis de l'effort de bridage exercé par le support de cornée 42 contre la couronne de bridage, ce qui s'avère parfois nécessaire pour obtenir un bridage optimum sans endommager le disque cornéoscléral.

Le support de cornée 42 possède à sa partie supérieure une tête 55 qui est identique à la tête 16 du mode de réalisation précédemment décrit en référence à la figure 1. De même, la chambre artificielle comporte un chapeau de bridage 56 qui est quasiment identique au chapeau de bridage 24 du mode de réalisation précédemment décrit en référence à la figure 1. La seule différence de ce chapeau de bridage par rapport à celui précédemment décrit réside dans l'absence d'ouverture 37 ménagée dans sa partie de liaison au pied 40, de telles ouvertures étant ici inutiles dans la mesure où l'opérateur n'agit pas directement sur le système d'actionnement du support de cornée.

## Revendications

1. Chambre artificielle pour un disque cornéoscléral en vue d'un prélèvement de greffon, comportant un pied (1 ; 40), un support de cornée (3 ; 42) porté par le pied (1 ; 40), ayant un axe vertical (4 ; 43) et possédant une tête (16 ; 55) présentant une face supérieure de support (18) sur laquelle débouche au moins un orifice d'alimentation (21), et une bague (1 ; 40) de bridage (25) du disque cornéoscléral sur le support de cornée (3 ; 42) dans laquelle la bague de bridage est portée par le pied, **caractérisée en ce que** la bague de bridage (25) est fixe tandis que le support de cornée (3 ; 42) est monté sur le pied (1 ; 40) pour coulisser suivant l'axe vertical (4 ; 43) du support de cornée (3 ; 42) et que des moyens d'actionnement (5, 6 ; 44, 46) sont prévus pour faire coulisser le support de cornée (3 ; 42).

2. Chambre artificielle selon la revendication 1, **caractérisée en ce que** les moyens d'actionnement du support de cornée (3) sont constitués par un système vis-écrou, le support de cornée (3) possédant une partie filetée (5) formant vis qui est arrêtée en rotation par rapport au pied (1) et qui coopère avec un écrou (6) monté sur le pied (1) en rotation libre sans translation selon l'axe (4) du support de cornée.

3. Chambre artificielle selon la revendication 1, **caractérisée en ce que** les moyens d'actionnement du support de cornée (42) sont constitués par un vérin double effet (44, 46).

4. Chambre artificielle selon la revendication 3, **caractérisée en ce que** le support de cornée (42) possède une partie inférieure (44) qui constitue la tige de son vérin d'actionnement.

5. Chambre artificielle selon l'une des revendications précédentes, **caractérisée en ce que** la bague de bridage (25) est montée de façon amovible sur le pied (1 ; 40).

6. Chambre artificielle selon la revendication **caractérisée en ce que** la fixation amovible de la bague de bridage (25) sur le pied (1 ; 40) est réalisée au moyen d'un système à baïonnette (35, 36).

7. Chambre artificielle selon l'une des revendications 5 et 6, prises en dépendance de la revendication 2, **caractérisée en ce que** la bague de bridage (25) est reliée au pied (1) par l'intermédaire d'une partie de liaison (34) qui entoure l'écrou d'actionnement (6) du support de cornée (3) et qui est ajourée pour permettre un actionnement manuel de cet écrou.

8. Chambre artificielle selon l'une quelconque des revendications précédentes, **caractérisée en ce que** la bague de bridage (25) est portée par le bord supérieur d'une couronne (27) qui coiffe la tête (16) du support de cornée (3 ; 42).

9. Chambre artificielle selon la revendication 8, **caractérisée en ce que** la couronne (27) présente un filetage extérieur (28) sur lequel est engagé un écrou (29) formant une butée réglable en hauteur pour un accessoire de kératotomie (30) venant coiffer la couronne de bridage (29).

10. Chambre artificielle selon la revendication 9, **caractérisée en ce que** l'accessoire de kératotomie est équipé d'un organe (32) de son verrouillage sur la couronne de bridage (24).

## Claims

1. An artificial chamber for a corneo-scleral disk for the purpose of taking a graft therefrom, the chamber comprising a stand (1; 40), a cornea support (3; 42) carried by the stand (1; 40), having a vertical axis (4; 43), and possessing a head (16; 55) having a supporting top face (18) into which there opens out at least one feed orifice (21), and a clamping ring (25) carried by the stand (1; 40) for clamping the corneo-scleral disk on the cornea support (3; 42), the chamber being **characterized in that** the clamping ring (25) is fixed, while the cornea support (3; 42) is mounted on the stand (1; 40) so as to slide along the vertical axis (4; 43) of the cornea support (3; 42) and actuator means (5, 6; 44, 46) are provided to slide the cornea support (3; 42).

2. An artificial chamber according to claim 1, **characterized in that** the actuator means of the cornea support (3) are constituted by a screw-and-nut system, the cornea support (3) possessing a screw-forming threaded portion (5) which is prevented from rotating relative to the stand (1) and which co-operates with a nut (6) mounted on the stand (1) to rotate freely without moving in translation along the axis (4) of the cornea support.

3. An artificial chamber according to claim 1, **characterized in that** the actuator means of the cornea support (42) are constituted by a double-acting actuator (44, 46).

4. An artificial chamber according to claim 3, **characterized in that** the cornea support (42) has a bottom portion (44) which constitutes the rod of its actuator.

5. An artificial chamber according to any preceding claim, **characterized in that** the clamping ring (25) is removably mounted on the stand (1; 40).

6. An artificial chamber according to claim 5, **characterized in that** the releasable fixing of the clamping ring (25) on the stand (1; 40) is provided by means of a bayonet system (35, 36).

7. An artificial chamber according to claim 5 or 6, as depending on claim 2, **characterized in that** the clamping ring (25) is connected to the stand (1) by means of a link portion (34) which surrounds the actuator nut (6) of the cornea support (3) and which is perforated so as to allow the nut to be actuated by hand.

8. An artificial chamber according to any preceding claim, **characterized in that** the clamping ring (25) is carried by the top edge of a collar (27) which covers the head (16) of the cornea support (3; 42).

9. An artificial chamber according to claim 8, **characterized in that** the collar (27) has an outside thread (28) on which a nut (29) is engaged which forms a stop of adjustable height for a keratotomy accessory (30) that covers the clamping collar (29).

10. An artificial chamber according to claim 9, **characterized in that** the keratotomy accessory is fitted with a member (32) for locking it to the clamping collar (24).

## Patentansprüche

1. Künstliche Kammer für eine Hornhaut-Sklera-Scheibe zur Entnahme eines Transplantats, umfassend einen Fuß (1; 40) getragenen Augenhomhaut-Träger (3;42), der eine vertikale Achse (4;43) hat und einen Kopf (16;55) besitzt, der eine obere Trägerfläche (18) aufweist, an der mindestens eine Versorgungsöffnung (21) mündet, sowie einen von dem Fuß (1;40) getragenen Spannring (25) zum Festspannen der Hornhaut-Sklera-Scheibe an dem Augenhornhaut-Träger (3; 42) **dadurch gekennzeichnet, dass** der Spannring (25) ortsfest ist, dass der Augenhornhaut-Träger (3; 42) derart am Fuß (1; 40) gelagert ist und entlang seiner vertikalen Achse (4; 43) verschoben werden kann, und dass Betätigungsmitteln (5, 6) um den Augenhornhaut-Träger (3; 42) zu schieben vorgesehen sind.

2. Künstliche Kammer nach Anspruch 1, **dadurch gekennzeichnet, dass** die Betätigungsmittel zur Betätigung des Augenhornhaut-Trägers (3) von einem Schraube-Mutter-System gebildet sind, wobei der Augenhornhaut-Träger (3) einen mit einem Gewinde versehenen Abschnitt (5) hat, der eine Schraube bildet, die gegenüber dem Fuß (1) drehfest ist und mit einer Mutter (6) zusammenwirkt, die an dem Fuß (1) frei drehbar ohne Translation entlang der Achse (4) des Augenhornhaut-Trägers gelagert ist.

3. Künstliche Kammer nach Anspruch 1, **dadurch gekennzeichnet, dass** die Betätigungsmittel zur Betätigung des Augenhornhaut-Trägers (42) von einem doppeltwirkenden Zylinder (44, 46) gebildet sind.

4. Künstliche Kammer nach Anspruch 3, **dadurch gekennzeichnet, dass** der Augenhornhaut-Träger (42) einen unteren Abschnitt (44) hat, der die Kolbenstange seines Betätigungszylinders bildet.

5. Künstliche Kammer nach einem der vorhergehenden Ansprüche, **dadurch gekennzeichnet, dass** der Spannring (25) lösbar am Fuß (1; 40) befestigt ist.

6. Künstliche Kammer nach Anspruch 5, **dadurch gekennzeichnet, dass** die lösbare Befestigung des Spannringes (25) am Fuß (1; 40) mittels eines Bajonettsystems (35, 36) erfolgt.

7. Künstliche Kammer nach einem der Ansprüche 5 und 6, in Abhängigkeit von Anspruch 2, **dadurch gekennzeichnet, dass** der Spannring (25) mit dem Fuß (1) über einen Verbindungsabschnitt (34) verbunden ist, der die Stellmutter (6) des Augenhornhaut-Trägers (3) umgibt und durchbrochen ist, um ein manuelles Verstellen dieser Mutter zu ermöglichen.

8. Künstliche Kammer nach einem der vorhergehenden Ansprüche, **dadurch gekennzeichnet, dass** der Spannring (25) von dem oberen Rand eines Kranzes (27) getragen wird, der den Kopf (16) des Augenhornhaut-Trägers (3; 42) abdeckt.

9. Künstliche Kammer nach Anspruch 8, **dadurch gekennzeichnet, dass** der Kranz (27) ein Außengewinde (28) hat, mit dem eine Mutter (29) in Eingriff steht, die einen höhenverstellbaren Anschlag für ein Keratotomie-Zubehör (30) bildet, das den Spannkranz (29) abdecken wird.

10. Künstliche Kammer nach Anspruch 9, **dadurch gekennzeichnet, dass** das Keratotomie-Zubehör mit einem Element (32) für seine Verriegelung am Spannkranz (24) versehen ist.
